# EUROPEAN PATENT APPLICATION

(11) **EP 3 088 404 A1**
(43) Date of publication of application: **02.11.2016**
(21) Application number: 14874403.0
(22) Date of filing: 22.12.2014
(51) Int. Cl.: C07F 9/6561, A61K 31/675, A61P 1/00, A61P 1/16, A61P 1/18, A61P 11/00, A61P 15/00, A61P 17/00, A61P 25/00, A61P 35/00, A61P 35/02, A61P 43/00

(54) **CRYSTAL (1) OF PYRAZINO[2,1-c][1,2,4]TRIAZINE COMPOUND**

(30) Priority: 25.12.2013 JP 2013267734
(71) Applicant: PRISM Pharma Co., Ltd., Kanagawa 226-8510 (JP)
(72) Inventor: KUSHIDA Ikuo, Tsukuba-shi Ibaraki 300-2635 (JP); SUGAYA Yukiko, Tsukuba-shi Ibaraki 300-2635 (JP); ODAGAMI Takenao, Yokohama-shi Kanagawa 226-8510 (JP); KOUJI Hiroyuki, Yokohama-shi Kanagawa 226-8510 (JP)
(74) Representative: Duckworth, Timothy John
(86) International application number: PCT/JP2014/083940
(87) International publication number: WO 2015/098857

(57) **Abstract**

Provided is a crystal of hydrate of 4-(((6S,9S)-1-(benzylcarbamoyl)-2,9-dimethyl-4,7-dioxo-8-(quinolin-8-ylmethyl)octahydro-1H-pyrazino[2,1-c][1,2,4]triazin-6-yl)methyl)phenyl dihydrogen phosphate, showing a diffraction peak at a diffraction angle (2θ±0.2°) of 15.9°, in powder X-ray diffraction.

## Description

### Technical Field

The present invention relates to a crystal of 4-(((6S,9S)-1-(benzylcarbamoyl)-2,9-dimethyl-4,7-dioxo-8-(quinolin-8-ylmethyl)octahydro-1H-pyrazino[2,1-c][1,2,4]triazin-6-yl)methyl)phenyl dihydrogen phosphate (hereinafter to be referred to as compound 1).

### Background Art

Compound 1 described in patent document 1 is a compound that inhibits the TCP4/β-Catenin transcription pathway by inhibiting CREB binding protein (CBP) in the Wnt signal transduction pathway, and is useful for the treatment of various carcinomass (e.g., lung cancer, breast cancer, stomach cancer, pancreatic cancer, liver cancer, uterine cancer, ovarian cancer, glioma, melanoma, rectal colon cancer, lymphoma, blood cancer), restenosis relating to angioplasty, angiogenesis abnormality, polycystic kidney, tuberous sclerosis, Alzheimer's disease, neurodegenerative diseases (e.g., glaucoma, macular degeneration, Parkinson's disease, Alzheimer's disease) and fibrosis (e.g., idiopathic pulmonary fibrosis).

### [Document List]

### Patent Documents

patent document 1: WO 2009/148192

### SUMMARY OF THE INVENTION

### Problems to be Solved by the Invention

In general, the property of a crystal of a compound utilizable as a pharmaceutical product exerts a marked influence on the bioavailability of a drug, purity of a drug substance, formulation of a preparation and the like.

Therefore, the problem of the present invention is to provide a crystal of compound 1 expected to be utilized as a drug substance of a pharmaceutical product.

### Means of Solving the Problems

The present inventors have conducted intensive studies in an attempt to solve the aforementioned problem and found a crystal of compound 1, and completed the present invention.

That is, the present invention provides the following [1] - [14].
[1] A crystal of 4-(((6S,9S)-1-(benzylcarbamoyl)-2,9-dimethyl-4,7-dioxo-8-(quinolin-8-ylmethyl)octahydro-1H-pyrazino[2,1-c][1,2,4]triazin-6-yl)methyl)phenyl dihydrogen phosphate or hydrate thereof.
[2] A crystal of hydrate of 4-(((6S,9S)-1-(benzylcarbamoyl)-2,9-dimethyl-4,7-dioxo-8-(quinolin-8-ylmethyl)octahydro-1H-pyrazino[2,1-c][1,2,4]triazin-6-yl)methyl)phenyl dihydrogen phosphate.
[3] The crystal of [2], showing diffraction peak at diffraction angle (2θ±0.2') of 15.9°, in powder X-ray diffraction.
[3A] The crystal of [2], showing diffraction peaks at diffraction angles (2θ±0.2°) of 15.9° and 18.0°, in powder X-ray diffraction.
[3B] The crystal of [2], showing diffraction peaks at diffraction angles (2θ±0.2°) of 15.9°, 18.0° and 23.2°, in powder X-ray diffraction.
[3C] The crystal of [2], showing diffraction peaks at diffraction angles (2θ±0.2°) of 3.2°, 15.9°, 18.0°, 23.2° and 24.8°, in powder X-ray diffraction.
[3D] The crystal of [2], showing diffraction peaks at diffraction angles (20±0.2°) of 3.2°, 8.2°, 11.0°, 14.3°, 15.9°, 18.0°, 19.3°, 23.2°, 24.8° and 28.5°, in powder X-ray diffraction.
[4] A crystal of hydrate of 4-(((6S,9S)-1-(benzylcarbamoyl)-2,9-dimethyl-4,7-dioxo-8-(quinolin-8-ylmethyl)octahydro-1H-pyrazino[2,1-c][1,2,4]triazin-6-yl)methyl)phenyl dihydrogen phosphate, showing peaks at chemical shifts (ppm) of 15.8, 56.4, 127.8, 157.4 and 168.6, in ¹³C solid-state NMR spectrum.
[5] A production method of a crystal of 4-(((6S,9S)-1-(benzylcarbamoyl)-2,9-dimethyl-4,7-dioxo-8-(quinolin-8-ylmethyl)octahydro-1H-pyrazino[2,1-c][1,2,4]triazin-6-yl)methyl)phenyl dihydrogen phosphate or hydrate thereof, comprising a step of crystallizing from a solvent selected from the group consisting of an alcohol solvent, water and a combination of these.
[6] A pharmaceutical composition comprising the crystal of any one of [1] - [3D] as an active ingredient.
[7] An antitumor agent comprising the crystal of any one of [1] - [3D] as an active ingredient.
[8] The antitumor agent of [7], wherein the tumor is lung cancer, breast cancer, stomach cancer, pancreatic cancer, liver cancer, uterine cancer, ovarian cancer, glioma, melanoma, rectal colon cancer, lymphoma or blood cancer.
[9] A method of preventing or treating tumor, comprising administering a pharmacologically effective amount of any one of the crystals of [1] - [3D] to a patient.
[10] The method of [9], wherein the tumor is lung cancer, breast cancer, stomach cancer, pancreatic cancer, liver cancer, uterine cancer, ovarian cancer, glioma, melanoma, rectal colon cancer, lymphoma or blood cancer.
[11] The crystal of any one of [1] - [3D], for use for the prophylaxis or treatment of tumor.
[12] The crystal of [11], wherein the tumor is lung cancer, breast cancer, stomach cancer, pancreatic cancer, liver cancer, uterine cancer, ovarian cancer, glioma, melanoma, rectal colon cancer, lymphoma or blood cancer.
[13] Use of the crystal of any one of [1] - [3D] in the production of an antitumor agent.
[14] Use of the crystal of [11], wherein the tumor is lung cancer, breast cancer, stomach cancer, pancreatic cancer, liver cancer, uterine cancer, ovarian cancer, glioma, melanoma, rectal colon cancer, lymphoma or blood cancer.

### Effect of the Invention

The crystal of compound 1 provided by the present invention has good property as a drug substance of a pharmaceutical product.

### Brief Description of the Drawings

Fig. 1 shows a powder X-ray diffraction pattern of crystal A obtained in Example 1, wherein the horizontal axis shows diffraction angle (2θ), and the vertical axis shows peak intensity.
Fig. 2 shows a powder X-ray diffraction pattern of crystal B obtained in Example 2, wherein the horizontal axis shows diffraction angle (2θ), and the vertical axis shows peak intensity.
Fig. 3 shows a powder X-ray diffraction pattern of crystal C obtained in Example 3, wherein the horizontal axis shows diffraction angle (2θ), and the vertical axis shows peak intensity.
Fig. 4 shows a powder X-ray diffraction pattern of crystal D obtained in Example 4, wherein the horizontal axis shows diffraction angle (2θ), and the vertical axis shows peak intensity.
Fig. 5 shows a powder X-ray diffraction pattern of an amorphous form of compound 1 obtained in Comparative Example 1, wherein the horizontal axis shows diffraction angle (2θ), and the vertical axis shows peak intensity.
Fig. 6 is a graph showing the hygroscopicity of crystal A obtained in Example 1, wherein the horizontal axis shows relative humidity (RH), and the vertical axis shows variation (%) in the mass.
Fig. 7 is a graph showing the thermal stability of crystal A obtained in Example 1.
Fig. 8 shows the ¹³C solid-state NMR spectrum of crystal A obtained in Example 1.

### Description of Embodiments

Preferable crystals in the present specification include
a crystal of hydrate of 4-(((6S,9S)-1-(benzylcarbamoyl)-2,9-dimethyl-4,7-dioxo-8-(quinolin-8-ylmethyl)octahydro-1H-pyrazino[2,1-c][1,2,4]triazin-6-yl)methyl)phenyl dihydrogen phosphate, showing a diffraction peak at diffraction angle (20±0.2') of 15.9°, in powder X-ray diffraction;
a crystal of hydrate of 4-(((6S,9S)-1-(benzylcarbamoyl)-2,9-dimethyl-4,7-dioxo-8-(quinolin-8-ylmethyl)octahydro-1H-pyrazino[2,1-c][1,2,4]triazin-6-yl)methyl)phenyl dihydrogen phosphate, showing diffraction peaks at diffraction angles (20±0.2') of 15.9° and 18.0°, in powder X-ray diffraction;
a crystal of hydrate of 4-(((6S,9S)-1-(benzylcarbamoyl)-2,9-dimethyl-4,7-dioxo-8-(quinolin-8-ylmethyl)octahydro-1H-pyrazino[2,1-c][1,2,4]triazin-6-yl)methyl)phenyl dihydrogen phosphate, showing diffraction peaks at diffraction angles (20±0.2') of 15.9°, 18.0° and 23.2°, in powder X-ray diffraction;
a crystal of hydrate of 4-(((6S,9S)-1-(benzylcarbamoyl)-2,9-dimethyl-4,7-dioxo-8-(quinolin-8-ylmethyl)octahydro-1H-pyrazino[2,1-c][1,2,4]triazin-6-yl)methyl)phenyl dihydrogen phosphate, showing diffraction peaks at diffraction angles (20±0.2') of 3.2°, 15.9°, 18.0°, 23.2° and 24.8°, in powder X-ray diffraction;
a crystal of hydrate of 4-(((6S,9S)-1-(benzylcarbamoyl)-2,9-dimethyl-4,7-dioxo-8-(quinolin-8-ylmethyl)octahydro-1H-pyrazino[2,1-c][1,2,4]triazin-6-yl)methyl)phenyl dihydrogen phosphate, showing diffraction peaks at diffraction angles (20±0.2°) of 3.2°, 8.2°, 11.0°, 14.3°, 15.9°, 18.0°, 19.3°, 23.2°, 24.8° and 28.5°, in powder X-ray diffraction;
a crystal of methanolate of 4-(((6S,9S)-1-(benzylcarbamoyl)-2,9-dimethyl-4,7-dioxo-8-(quinolin-8-ylmethyl)octahydro-1H-pyrazino[2,1-c][1,2,4]triazin-6-yl)methyl)phenyl dihydrogen phosphate, showing a diffraction peak at diffraction angle (2θ±0.2°) of 10.3°;
a crystal of methanolate of 4-(((6S,9S)-1-(benzylcarbamoyl)-2,9-dimethyl-4,7-dioxo-8-(quinolin-8-ylmethyl)octahydro-1H-pyrazino[2,1-c][1,2,4]triazin-6-yl)methyl)phenyl dihydrogen phosphate, showing diffraction peaks at diffraction angles (2θ±0.2°) of 10.3° and 10.8°, in powder X-ray diffraction;
a crystal of methanolate of 4-(((6S,9S)-1-(benzylcarbamoyl)-2,9-dimethyl-4,7-dioxo-8-(quinolin-8-ylmethyl)octahydro-1H-pyrazino[2,1-c][1,2,4]triazin-6-yl)methyl)phenyl dihydrogen phosphate, showing diffraction peaks at diffraction angles (20±0.2°) of 10.3°, 10.8° and 22.9°, in powder X-ray diffraction;
a crystal of methanolate of 4-(((6S,9S)-1-(benzylcarbamoyl)-2,9-dimethyl-4,7-dioxo-8-(quinolin-8-ylmethyl)octahydro-1H-pyrazino[2,1-c][1,2,4]triazin-6-yl)methyl)phenyl dihydrogen phosphate, showing diffraction peaks at diffraction angles (20±0.2°) of 10.3°, 10.8°, 14.4° and 22.9°, in powder X-ray diffraction;
a crystal of methanolate of 4-(((6S,9S)-1-(benzylcarbamoyl)-2,9-dimethyl-4,7-dioxo-8-(quinolin-8-ylmethyl)octahydro-1H-pyrazino[2,1-c][1,2,4]triazin-6-yl)methyl)phenyl dihydrogen phosphate, showing diffraction peaks at diffraction angles (20±0.2°) of 10.3°, 10.8°, 14.4°, 22.9° and 23.3°, in powder X-ray diffraction;
a crystal of methanolate of 4-(((6S,9S)-1-(benzylcarbamoyl)-2,9-dimethyl-4,7-dioxo-8-(quinolin-8-ylmethyl)octahydro-1H-pyrazino[2,1-c][1,2,4]triazin-6-yl)methyl)phenyl dihydrogen phosphate, showing diffraction peaks at diffraction angles (20±0.2') of 7.9°, 10.3°, 10.8°, 13.1°, 14.4°, 20.9°, 22.9°, 23.3°, 24.7° and 26.5°, in powder X-ray diffraction;
a crystal of anhydride of 4-(((6S,9S)-1-(benzylcarbamoyl)-2,9-dimethyl-4,7-dioxo-8-(quinolin-8-ylmethyl)octahydro-1H-pyrazino[2,1-c][1,2,4]triazin-6-yl)methyl)phenyl dihydrogen phosphate, showing a diffraction peak at diffraction angle (2θ±0.2°) of 8.3°;
a crystal of anhydride of 4-(((6S,9S)-1-(benzylcarbamoyl)-2,9-dimethyl-4,7-dioxo-8-(quinolin-8-ylmethyl)octahydro-1H-pyrazino[2,1-c][1,2,4]triazin-6-yl)methyl)phenyl dihydrogen phosphate, showing diffraction peaks at diffraction angles (20±0.2°) of 8.3° and 18.6°, in powder X-ray diffraction;
a crystal of anhydride of 4-(((6S,9S)-1-(benzylcarbamoyl)-2,9-dimethyl-4,7-dioxo-8-(quinolin-8-ylmethyl)octahydro-1H-pyrazino[2,1-c][1,2,4]triazin-6-yl)methyl)phenyl dihydrogen phosphate, showing diffraction peaks at diffraction angles (20±0.2') of 8.3°, 13.0° and 18.6°, in powder X-ray diffraction;
a crystal of anhydride of 4-(((6S,9S)-1-(benzylcarbamoyl)-2,9-dimethyl-4,7-dioxo-8-(quinolin-8-ylmethyl)octahydro-1H-pyrazino[2,1-c][1,2,4]triazin-6-yl)methyl)phenyl dihydrogen phosphate, showing diffraction peaks at diffraction angles (20±0.2°) of 8.3°, 13.0°, 15.8° and 18.6°, in powder X-ray diffraction;
a crystal of anhydride of 4-(((6S,9S)-1-(benzylcarbamoyl)-2,9-dimethyl-4,7-dioxo-8-(quinolin-8-ylmethyl)octahydro-1H-pyrazino[2,1-c][1,2,4]triazin-6-yl)methyl)phenyl dihydrogen phosphate, showing diffraction peaks at diffraction angles (2θ±0.2°) of 8.3°, 12.7°, 13.0°, 15.8° and 18.6°, in powder X-ray diffraction;
a crystal of anhydride of 4-(((6S,9S)-1-(benzylcarbamoyl)-2,9-dimethyl-4,7-dioxo-8-(quinolin-8-ylmethyl)octahydro-1H-pyrazino[2,1-c][1,2,4]triazin-6-yl)methyl)phenyl dihydrogen phosphate, showing diffraction peaks at diffraction angles (20±0.2') of 8.3°, 12.7°, 13.0°, 15.3°, 15.8°, 17.1°, 18.6°, 19.6°, 23.8° and 24.9°, in powder X-ray diffraction;
a crystal of dimethyl sulfoxide solvate of 4-(((6S,9S)-1-(benzylcarbamoyl)-2,9-dimethyl-4,7-dioxo-8-(quinolin-8-ylmethyl)octahydro-1H-pyrazino[2,1-c][1,2,4]triazin-6-yl)methyl)phenyl dihydrogen phosphate, showing a diffraction peak at diffraction angle (20±0.2') of 18.4°;
a crystal of dimethyl sulfoxide solvate of 4-(((6S,9S)-1-(benzylcarbamoyl)-2,9-dimethyl-4,7-dioxo-8-(quinolin-8-ylmethyl)octahydro-1H-pyrazino[2,1-c][1,2,4]triazin-6-yl)methyl)phenyl dihydrogen phosphate, showing diffraction peaks at diffraction angles (20±0.2') of 15.9° and 18.4°, in powder X-ray diffraction;
a crystal of dimethyl sulfoxide solvate of 4-(((6S,9S)-1-(benzylcarbamoyl)-2,9-dimethyl-4,7-dioxo-8-(quinolin-8-ylmethyl)octahydro-1H-pyrazino[2,1-c][1,2,4]triazin-6-yl)methyl)phenyl dihydrogen phosphate, showing diffraction peaks at diffraction angles (20±0.2°) of 15.9°, 18.4° and 25.5°, in powder X-ray diffraction;
a crystal of dimethyl sulfoxide solvate of 4-(((6S,9S)-1-(benzylcarbamoyl)-2,9-dimethyl-4,7-dioxo-8-(quinolin-8-ylmethyl)octahydro-1H-pyrazino[2,1-c][1,2,4]triazin-6-yl)methyl)phenyl dihydrogen phosphate, showing diffraction peaks at diffraction angles (20±0.2°) of 15.9°, 18.4°, 20.9° and 25.5°, in powder X-ray diffraction;
a crystal of dimethyl sulfoxide solvate of 4-(((6S,9S)-1-(benzylcarbamoyl)-2,9-dimethyl-4,7-dioxo-8-(quinolin-8-ylmethyl)octahydro-1H-pyrazino[2,1-c][1,2,4]triazin-6-yl)methyl)phenyl dihydrogen phosphate, showing diffraction peaks at diffraction angles (2θ±0.2°) of 15.9°, 18.4°, 20.9°, 24.8° and 25.5°, in powder X-ray diffraction;
a crystal of dimethyl sulfoxide solvate of 4-(((6S,9S)-1-(benzylcarbamoyl)-2,9-dimethyl-4,7-dioxo-8-(quinolin-8-ylmethyl)octahydro-1H-pyrazino[2,1-c][1,2,4]triazin-6-yl)methyl)phenyl dihydrogen phosphate, showing diffraction peaks at diffraction angles (20±0.2') of 15.9°, 17.2°, 18.4°, 20.9°, 21.9°, 24.2°, 24.6°, 24.8°, 25.5° and 28.5°, in powder X-ray diffraction, and the like.

In general, the diffraction angle (2θ) in powder X-ray diffraction produces an error within the range of ±0.2°. Therefore, the value of the above-mentioned diffraction angle needs to be understood to include numerical values within the range of about ±0.2°. Therefore, not only the crystals showing peaks at completely identical diffraction angles in powder X-ray diffraction, but also crystals showing peaks at diffraction angles different only by about ±0.2° are also encompassed in the present invention.

Therefore, in the present specification, for example, "showing a diffraction peak at a diffraction angle (20±0.2°) of 15.9°" means that it "shows a diffraction peak at a diffraction angle (2θ) of 15.7° - 16.1°", and the same applies to other diffraction angles.

### Production method of a crystal of compound 1 or solvate thereof

A crystal of compound 1 or solvate thereof can be produced by dissolving compound 1 or a salt thereof in a solvent by heating, and crystallizing same by cooling under stirring. Examples of the solvate include hydrate, methanolate, dimethyl sulfoxide solvate and the like. The solvate of compound 1 is preferably a hydrate.

Examples of the crystal of compound 1 or solvate thereof include below-mentioned crystal A - D. The obtained crystal of compound 1 or solvate thereof varies depending on the crystallization conditions such as the kind of the solvent and the like. Those of ordinary skill in the art can appropriately produce a desired crystal by reference to the below-mentioned production method of crystals.

Compound 1 or a salt thereof to be used for crystallization may have any form, and may be a hydrate or an anhydride, may be amorphous or crystalline (including one composed of plural polycrystallines), and may be a mixture of these.

The solvent used for crystallization includes, for example, alcohol solvents such as methanol, ethanol, isopropanol, 1-propanol and the like; acetonitrile; dimethyl sulfoxide (DMSO); amide solvents such as N,N-dimethylformamide (DMF) and the like; ester solvents such as methyl acetate, ethyl acetate, isopropyl acetate and the like; saturated hydrocarbon solvents such as hexane, heptane and the like; ketone solvents such as acetone, methylethyl ketone and the like; ether solvents such as t-butyl methyl ether, tetrahydrofuran (THF) and the like; dichloromethane and water. These solvents may be used alone or a mixture of two or more kinds thereof may be used.

The amount of the solvent to be used can be appropriately selected wherein an amount capable of dissolving compound 1 or a salt thereof by heating or an amount that enables stirring of a suspension obtained by adding a solvent to compound 1 or a salt thereof is the lower limit, and an amount free of a marked decrease in the yield of crystal is the upper limit.

The crystal obtained by the above-mentioned method has a form of a single crystal. This crystal form is stable, does not easily transit to other crystal form or amorphous form, has good property and is also suitable for formulation.

In crystallization, a seed crystal (crystal of desired compound 1 and the like) may or may not be added. While the temperature at which a seed crystal is added is not particularly limited, it is preferably 0°C - 60°C.

As the temperature at which compound 1 or a salt thereof is dissolved by heating, a temperature at which compound 1 is dissolved can be appropriately selected according to the solvent. Preferred is a temperature range from a temperature at which a solvent used for crystallization starts refluxing to 50°C, and more preferred is 55°C - 65°C.

Since cooling during crystallization may afford a crystal with different form (polymorphism) when the cooling is rapid. Therefore, cooling is desirably performed after appropriately adjusting the cooling rate in consideration of the quality of crystal and influence on the particle size and the like. Cooling at 5°C - 40°C/h is preferable, and cooling at a rate of 5°C - 25°C/h is more preferable.

While the final crystallization temperature can be appropriately selected according to the desired object such as yield, quality and the like of the crystal, it is preferably-25°C - 30°C.

The object crystal can be obtained by separating the crystal obtained by crystallization by a general filtration operation, washing the filtered crystal with a solvent as necessary, and drying same. As the solvent to be used for washing the crystal, those similar to the solvents used for crystallization can be used. Examples of the solvent to be used for washing include acetone, methylethyl ketone, ethyl acetate, and t-butyl methyl ether.

The crystal separated by the filtration operation can be dried by leaving in the air or a nitrogen stream as appropriate, or by heating.

The drying time can be selected as appropriate as the time necessary for the residual solvent to become less than a given amount, and according to the production amount, dryer, drying temperature and the like. Drying can be performed under ventilation or under reduced pressure. The depressurization level can be appropriately selected according to the production amount, dryer, drying temperature and the like. The obtained crystal can also be left in the air as necessary after drying.

The above-mentioned crystal can also be produced by, in the above-mentioned production method of compound 1, performing the production method of the crystal of compound 1 or solvate thereof sequentially after the synthesis of compound 1.

First, an amorphous form of compound 1 is explained. Compound 1 can be produced by synthesize according to the method described in patent document 1. When crystal B is heated in an oven at 50°C overnight, it changes into an amorphous form.

Crystals A - D are now explained below.

Crystal A is a crystal of a hydrate of compound 1, and has a melting point of 164°C. Crystal A can be produced by resuspending in or crystallizing from water, ethyl acetate, methyl acetate, dichloromethane, or an aqueous solution containing 50 mass % of water. Such crystal is a more preferable embodiment.

Crystal B is a crystal of a methanolate of compound 1, and has a melting point of 136°C. Crystal B can be produced by resuspending in or crystallizing from a solvent containing methanol or methanol.

Crystal C is a crystal of an anhydride of compound 1, and has a melting point of 172°C. Crystal C can be produced by resuspending in or crystallizing from a solvent such as acetone, acetonitrile, isopropanol, tetrahydrofuran and the like.

Crystal D is a crystal of a dimethyl sulfoxide solvate of compound 1. Crystal D can be produced by resuspending in or crystallizing from DMSO.

The crystal of compound 1 or solvate thereof can be formulated according to a conventional method. Examples of the dosage form of formulation include oral preparation (tablet, granule, powder, capsule, syrup etc.), injection (for intravenous administration, intramuscular administration, subcutaneous administration, intraperitoneal administration etc.), and external preparation (transdermal absorption preparation (ointment, adhesive preparation etc.), eye drop, nasal drop, suppository etc.).

When an oral solid preparation (tablet, granule, powder, capsule etc.) is produced, additives such as excipient, binder, disintegrant, lubricant, colorant and the like are added as necessary to the crystal of compound 1 or solvate thereof, and an oral solid preparation is produced by a conventional method. When an oral solid preparation is produced, coating may be applied as necessary.

Examples of the excipient include lactose, cornstarch, crystalline cellulose and the like, and examples of the binder include hydroxypropylcellulose, hydroxypropylmethylcellulose and the like. Examples of the disintegrant include calcium carboxymethylcellulose, croscarmellose sodium and the like, and examples of the lubricant include magnesium stearate, calcium stearate and the like. Examples of the colorant include titanium oxide and the like, and examples of the coating agent include hydroxypropylcellulose, hydroxypropylmethylcellulose, methylcellulose and the like. Usable additives are not limited to these.

These oral solid preparations can generally contain 0.001 - 99.5 mass%, preferably 0.01 - 90 mass% and the like, of the crystal of compound 1.

When an injection (for intravenous administration, intramuscular administration, subcutaneous administration, intraperitoneal administration etc.) is produced, pH adjuster, buffering agent, suspending agent, solubilizing agent, antioxidant, preservative (antiseptic), isotonicity agent and the like are added as necessary to the crystal of compound 1, and injection can be produced by a conventional method. The injection may be formed as a use-time dissolution type freeze-dry preparation by freeze-drying.

As the pH adjuster and buffering agent, organic acid or inorganic acid and/or a salt thereof and the like can be used. As the suspending agent, methylcellulose, polysorbate 80, sodium carboxymethylcellulose and the like can be used; as the solubilizing agent, polysorbate 80, polyoxyethylene sorbitan monolaurate and the like can be used. As the antioxidant, α-tocopherol and the like can be used; as the preservative, methyl p-hydroxybenzoate, ethyl parahydroxybenzoate and the like can be used; as the isotonicity agent, glucose, sodium chloride, mannitol and the like can be used. The pH adjuster, buffering agent, suspending agent, solubilizing agent, antioxidant, preservative (antiseptic), and isotonicity agent are not limited to these.

These injections can generally contain 0.000001 - 99.5 mass %, preferably 0.00001 - 90 mass % and the like of the crystal of compound 1 relative to the total mass of the injection.

When an external preparation is produced, a base starting material is added to the crystal of compound 1 and, for example, preservative, stabilizer, pH adjuster, antioxidant, colorant and the like are added as necessary, and an external preparation can be produced by a conventional method.

As the base starting material to be used, various starting materials generally used for pharmaceutical products, quasi-drugs, cosmetics and the like can be used. Specifically, for example, starting materials such as animal and vegetable oils, mineral oil, ester oil, waxes, emulsifier, higher alcohols, fatty acids, silicon oil, surfactant, phospholipids, alcohols, polyvalent alcohols, water-soluble polymers, clay minerals, purified water and the like can be mentioned.

These external preparations can generally contain 0.000001 - 99.5 mass%, preferably 0.00001 - 90 mass% and the like of the crystal of compound 1.

While the dose of the crystal of compound 1 or solvate thereof varies depending on the level of symptom, age, sex, body weight, administration form, kind of salt, specific kind of the disease and the like, in the case of an adult, generally about 30 µg - 10 g, preferably 100 µg - 5 g, further preferably 100 µg - 1 g, is administered per day by oral administration, and about 30 µg - 1 g, preferably 100 µg - 500 mg, further preferably 100 µg - 300 mg, is administered per day by injection administration, each in one to several portions. Examples

The present invention is explained in detail in the following by referring to Comparative Examples and Examples, which are not to be construed as limitative. The "room temperature" is a temperature within the range of 1 - 30°C.

The chemical shift of ¹H-NMR (proton nuclear magnetic resonance) spectrum was recorded in δ unit (ppm) to tetramethylsilane, and the coupling constant was recorded in hertz (Hz). The abbreviations of splitting patterns are as follows. s: singlet, d: doublet, t: triplet, q: quartette, m: multiplet, brs: broad singlet, brd: broad doublet.

### Comparative Example 1

### Preparation of amorphous compound 1

According to the production method described in patent document 1, compound 1 was obtained as a solid. ¹H-NMR (600 MHz, METHANOL-d₄) δ (ppm): 1.15 (d, J=6 Hz, 3H), 2.65 (s, 3H), 3.12 (d, J=18 Hz, 1H), 3.35 (d, J=7 Hz, 2H), 3.48 (d, J=18 Hz, 1H), 4.15 (m, 1H), 4.32 (d, J=15 Hz, 1H), 4.40 (d, J=15 Hz, 1H), 5.33 (d, J=16 Hz, 1H), 5.41 (d, J=16 Hz, 1H), 5.44 (d, J=7 Hz, 1H), 5.64 (d, J=10 Hz, 1H), 7.07 (dd, J=9, 1 Hz, 2H), 7.15 (d, J=9 Hz, 2H), 7.24 (t, J=7 Hz, 1H), 7.27 (d, J=7 Hz, 2H), 7.34 (t, J=8 Hz, 2H), 7.55 (dd, J=8, 4 Hz, 1H), 7.60 (brd, J=6 Hz, 1H), 7.62 (dd, J=8, 7 Hz, 1H), 7.88 (dd, J=8, 1 Hz, 1H), 8.38 (dd, J=8, 2 Hz, 1H), 8.90 (dd, J=4, 2 Hz, 1H).

### Example 1

### Preparation of a crystal of hydrate of compound 1 (crystal A)

To compound 1 (149.72 mg) obtained in Comparative Example 1 was added a ethanol/water mixed solution (volume ratio 1:1, 4.0 mL) and the mixture was stirred with heating. As a result, it was completely dissolved at about 93°C. The mixture was allowed to slowly cool to room temperature, and precipitation of a solid was confirmed. The precipitate was collected by filtration through a glass filter, washed with heptane, and air dried at 60°C to give the title crystal (111.35 mg) as a white solid.

### Example 2

### Preparation of a crystal of methanolate of compound 1 (crystal B)

Crystal A (100 mg) was dissolved in methanol (1000 mL), and the mixture was stirred at room temperature overnight, and heated under reduced pressure at 45°C to evaporate the solvent to give the title crystal.

### Example 3

### Preparation of crystal of anhydride of compound 1 (crystal C)

Crystal A (100 mg) was dissolved in ethanol (1000 mL), and the mixture was stirred at room temperature overnight. The precipitated solid was collected by filtration and dried at 45°C for 12 hr to give the title crystal.

### Example 4

### Preparation of crystal of dimethyl sulfoxide solvate of compound 1 (crystal D)

DMSO (1 mL) was added to crystal B (2000 mg), and the mixture was stirred at 50°C overnight. The solution was cooled to room temperature and the precipitated solid was collected by filtration to give the title crystal.

### Experimental Example 1: Measurement of powder X-ray diffraction

Respective crystals obtained in Examples 1 - 4 were placed on a sample table of a powder X-ray diffraction apparatus, and measurement was performed under the following conditions.

### (measurement conditions)

apparatus: Rigaku MiniFlex II Desktop X-ray Diffractometer
X-ray used: Cu Kα ray
detector: scintillation counter
tube voltage: 30 kV
tube electric current: 15 mA
Kβ filter: nickel filter
divergence slit: 1.25°
scattering slit: 1.25°
receiving slit: 0.3 mm
soller slit: 5° (divergence angle)
scan rate: 5°/min
sampling interval: 0.02°
scan range: 3° - 36°
sample holder: aluminum holder

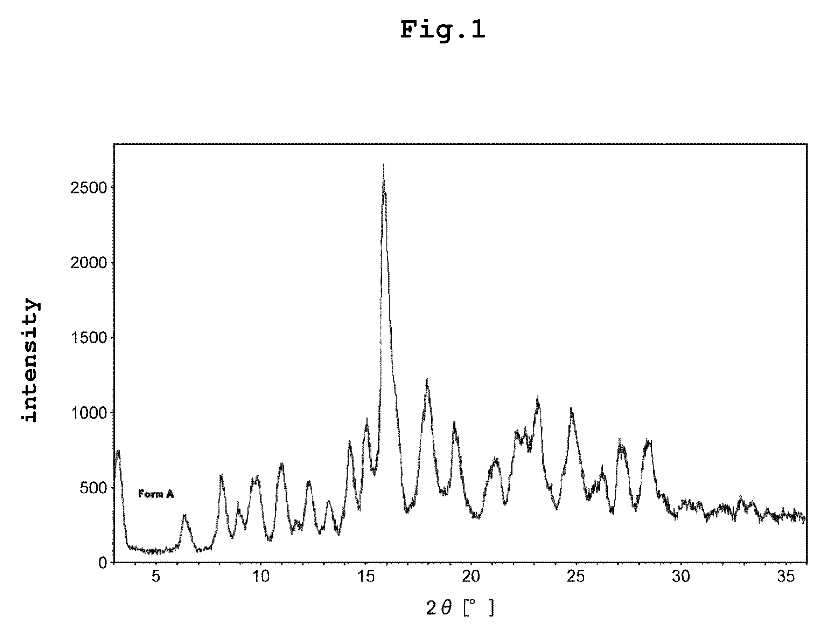

The powder X-ray crystal diffraction of crystal A obtained in Example 1 was measured, and the diffraction pattern shown in Fig. 1 and Table 1 was obtained.

**Table 1**

| 2θ [°] | peak intensity [counts] |
|---|---|
| 3.2 | 650 |
| 6.4 | 231 |
| 8.2 | 484 |
| 8.9 | 151 |
| 9.6 | 369 |
| 9.9 | 358 |
| 11.0 | 464 |
| 11.7 | 43 |
| 12.3 | 323 |
| 13.3 | 202 |
| 14.3 | 481 |
| 15.1 | 415 |
| 15.9 | 2027 |
| 18.0 | 792 |
| 19.3 | 477 |
| 21.2 | 311 |
| 22.2 | 287 |
| 22.6 | 396 |
| 23.2 | 695 |
| 24.8 | 603 |
| 25.9 | 102 |
| 26.3 | 226 |
| 27.1 | 419 |
| 28.5 | 439 |
| 30.1 | 86 |
| 30.9 | 74 |
| 32.9 | 123 |
| 33.4 | 99 |
| 34.2 | 55 |

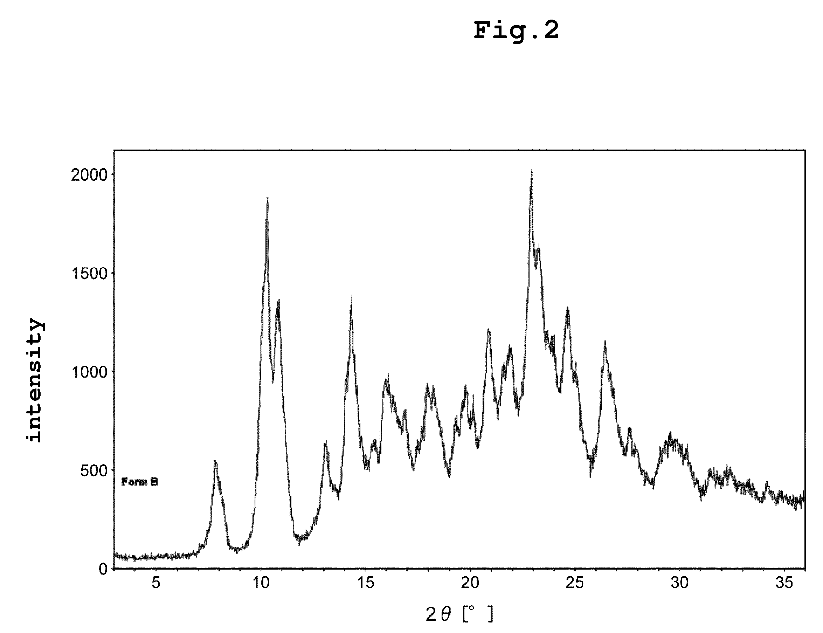

The powder X-ray crystal diffraction of crystal B obtained in Example 2 was measured, and the diffraction pattern shown in Fig. 2 and Table 2 was obtained.

**Table 2**

| 2θ [°] | peak intensity [counts] |
|---|---|
| 7.9 | 467 |
| 10.3 | 1716 |
| 10.8 | 1169 |
| 13.1 | 482 |
| 14.4 | 867 |
| 15.5 | 85 |
| 16.0 | 324 |
| 16.3 | 305 |
| 16.9 | 127 |
| 189.0 | 397 |
| 18.3 | 386 |
| 19.3 | 219 |
| 19.8 | 323 |
| 20.2 | 176 |
| 20.9 | 469 |
| 21.6 | 237 |
| 21.9 | 277 |
| 22.9 | 1009 |
| 23.3 | 762 |
| 24.7 | 483 |
| 25.1 | 264 |
| 26.5 | 570 |
| 26.9 | 321 |
| 27.6 | 144 |
| 29.2 | 126 |
| 29.6 | 215 |
| 29.9 | 192 |
| 31.5 | 74 |
| 34.2 | 82 |

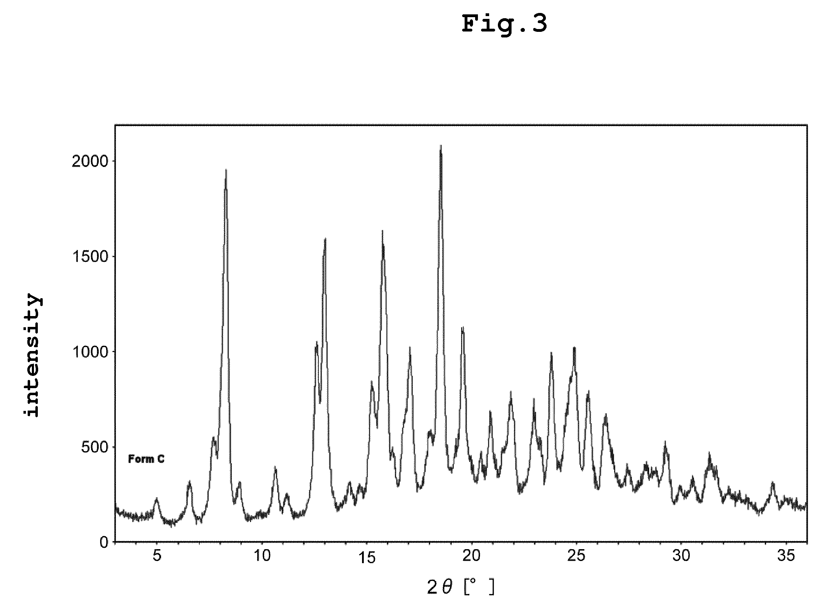

The powder X-ray crystal diffraction of crystal C obtained in Example 3 was measured, and the diffraction pattern shown in Fig. 3 and Table 3 was obtained.

**Table 3**

| 2θ [°] | peak intensity [counts] |
|---|---|
| 5.0 | 111 |
| 6.6 | 196 |
| 7.7 | 390 |
| 8.3 | 1788 |
| 9.0 | 133 |
| 10.7 | 249 |
| 11.2 | 109 |
| 12.7 | 905 |
| 13.0 | 1407 |
| 14.2 | 108 |
| 15.3 | 606 |
| 15.8 | 1281 |
| 16.8 | 339 |
| 17.1 | 686 |
| 18.0 | 252 |
| 18.6 | 1710 |
| 19.6 | 767 |
| 20.5 | 129 |
| 20.9 | 308 |
| 21.9 | 441 |
| 23.0 | 371 |
| 23.3 | 202 |
| 23.8 | 602 |
| 24.7 | 371 |
| 24.9 | 643 |
| 25.6 | 420 |
| 26.4 | 353 |
| 27.5 | 93 |
| 28.3 | 102 |
| 28.8 | 135 |
| 29.3 | 230 |
| 30.0 | 76 |
| 30.6 | 104 |
| 31.4 | 217 |
| 31.7 | 151 |
| 32.3 | 52 |
| 34.4 | 125 |

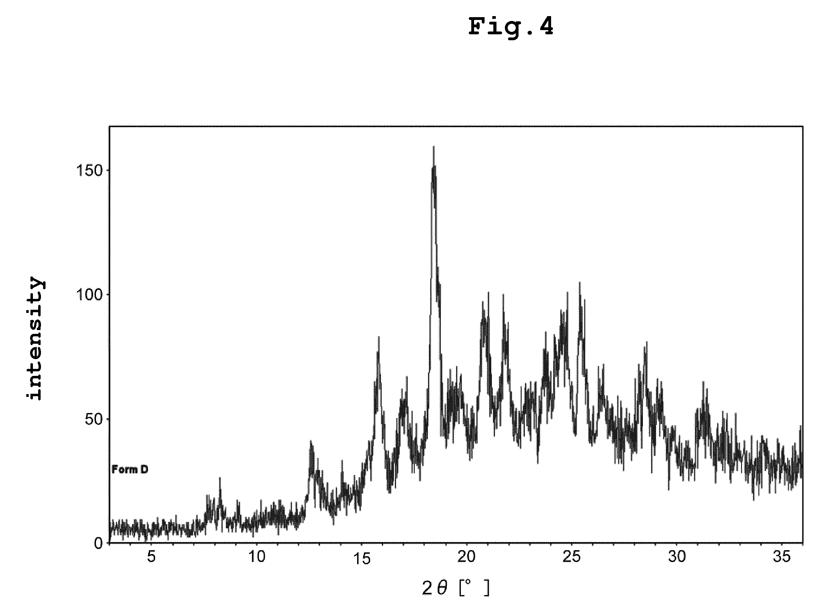

The powder X-ray crystal diffraction of crystal D obtained in Example 4 was measured, and the diffraction pattern shown in Fig. 4 and Table 4 was obtained.

**Table 4**

| 2θ [°] | peak intensity [counts] |
|---|---|
| 7.7 | 9 |
| 8.0 | 9 |
| 8.3 | 14 |
| 9.1 | 5 |
| 12.6 | 24 |
| 12.9 | 17 |
| 14.1 | 10 |
| 15.4 | 17 |
| 15.9 | 52 |
| 16.9 | 26 |
| 17.2 | 28 |
| 18.4 | 112 |
| 19.2 | 13 |
| 19.7 | 21 |
| 20.9 | 40 |
| 21.9 | 32 |
| 22.8 | 15 |
| 23.7 | 23 |
| 24.2 | 32 |
| 24.6 | 34 |
| 24.8 | 35 |
| 25.5 | 48 |
| 26.3 | 18 |
| 26.5 | 21 |
| 28.2 | 20 |
| 28.5 | 34 |
| 29.1 | 20 |
| 29.3 | 17 |
| 29.8 | 10 |
| 31.0 | 14 |
| 31.5 | 18 |
| 32.4 | 8 |
| 32.8 | 8 |
| 34.2 | 13 |

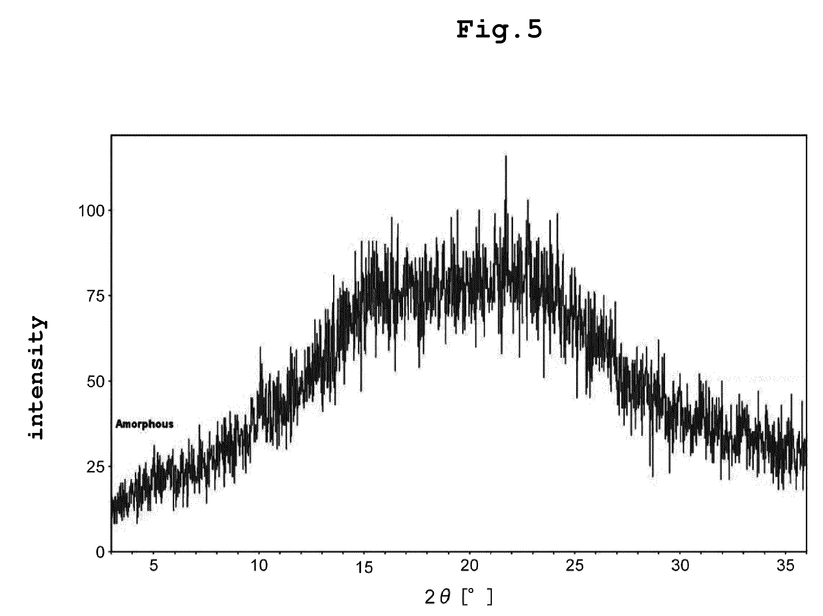

Similarly, the powder X-ray diffraction pattern of the solid obtained in Comparative Example 1 is shown in Fig. 5. From Fig. 5, it was confirmed that the solid obtained in Comparative Example 1 was amorphous.

### Experimental Example 2: hygroscopicity evaluation

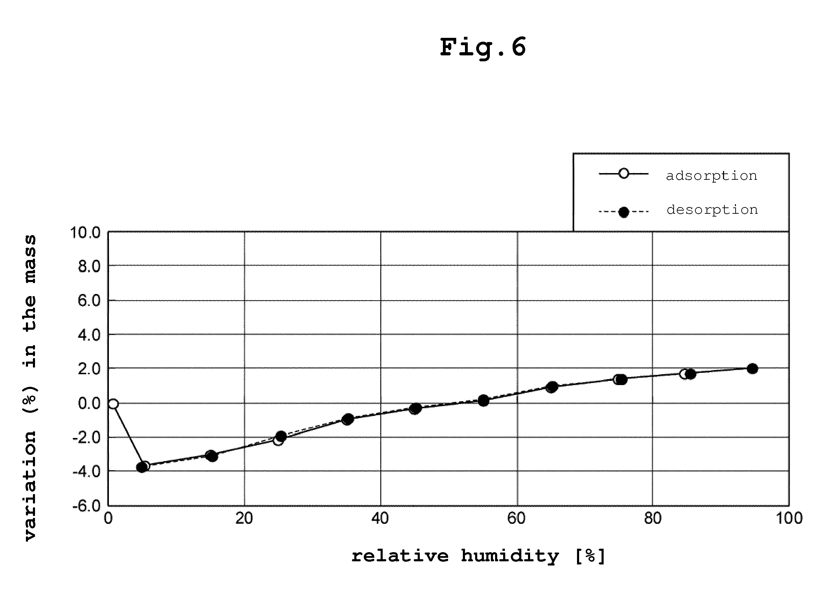

The hygroscopicity of crystal A obtained in Example 1 was evaluated by a dynamic water adsorption measuring apparatus. A sample mounting part of the apparatus was maintained at 25°C, and the relative humidity (RH) was set stepwisely within the range of 5 - 95%RH. The humidity was controlled by changing the relative flow of 0%RH dry nitrogen and 100%RH humidified nitrogen. The sample weight was confirmed at for 2 min intervals by a micro balance, and the humidity was sequentially changed at the time point when the weight shift range for 5 min was below 0.01%. The results are shown in Fig. 6.

As shown in Fig. 6, the crystal obtained in Example 1 showed low hygroscopicity within the relative humidity range of 35 - 75%RH, and the weight change was about 2.5% at the maximum.

### Experimental Example 3: Evaluation of thermal stability

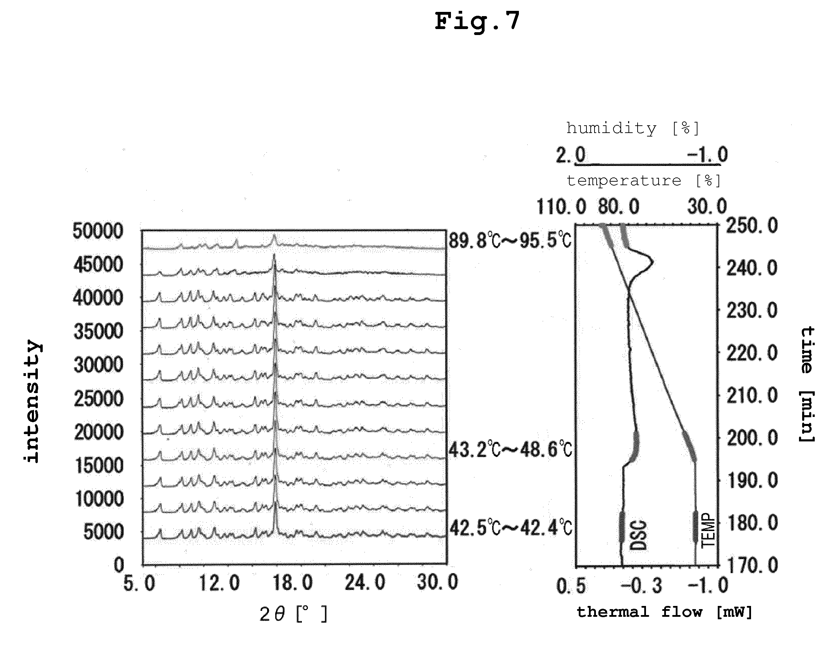

Using X-ray diffraction - differential scanning simultaneous calorimetry apparatus (X-ray-DSC), whether the crystal form of crystal A obtained in Example 1 changes upon heating was observed. The results are shown in Fig. 7.

The right graph of Fig. 7 shows thermal behavior when crystal A obtained in Example 1 was heated from 40°C to 100°C at 1°C/min, and the left spectrum of Fig. 7 shows time-course changes in the powder X ray crystal diffraction pattern of crystal A due to the heating. In the thermal behavior on the right in Fig. 7, an endothermic peak suggesting desorption of hydrated water in crystal A at 43°C - 48°C; however, the diffraction pattern did not change at that time. When the temperature was around 90°C, the weight of crystal A decreased, and the powder X-ray diffraction pattern changed clearly. Crystal A is thermally stable.

### Experimental Example 4: Evaluation of solid stability

The solid obtained in Example 1 or Comparative Example 1 was preserved for 3 months under the following 3 kinds of preservation conditions. The absorbance at wavelength 226 nm was measured by high performance liquid chromatography (HPLC), and the content of impurity contained in the crystal was measured every other month.

### (Preservation conditions)

(1) -20°C
(2) 40°C/75%RH
(3) 60°C

From the peak area of the obtained chromatogram, a value obtained by dividing the total area of respective peaks other than the peak showing compound 1, by the total area of all the obtained peaks, from the peaks contained in the above-mentioned solid, was taken as impurity content (%), and is shown in Table 5. By comparison of the impurity contents 3 months later, preservation under the condition of 40°C, 75%RH resulted in an about 4% increase in the impurity content of the amorphous form of Comparative Example 1 and an about 1.6% increase in the crystal of Example 1, as compared to preservation under the condition of -20°C. Even when preserved under the condition of 60°C, Comparative Example 1 showed an increase by not less than 1.1% and Example 1 showed an increase by not more than 0.5% as compared to preservation under the condition of -20°C. Therefore, the crystal of Example 1 was more stable than the amorphous form of Comparative Example 1 under high temperature conditions and high humidity conditions, and had preferable property as a drug substance for pharmaceutical products.

**Table 5**

| | preservation conditions | impurity content (%) | | |
|---|---|---|---|---|
| | | 1 month later | 2 months later | 3 months later |
| Comparative Example 1 | -20°C | 2.46 | 2.60 | 2.57 |
| | 40°C/75%RH | 3.35 | 4.62 | 6.51 |
| | 60°C | 3.10 | 3.41 | 3.68 |
| Example 1 | -20°C | 0.24 | 0.17 | 0.24 |
| | 40°C/75%RH | 0.65 | 1.02 | 1.80 |
| | 60°C | 0.53 | 0.59 | 0.72 |

### Experimental Example 5: Measurement of ¹³C solid-state NMR spectrum

The ¹³C solid-state NMR spectrum in Example 1 was measured under the following conditions.
apparatus used: Avance 400 MHz (manufactured by BRUKER) 7 mm-CPMAS probe (manufactured by BRUKER)
measured nucleus: ¹³C (resonance frequency: 100.6248425 MHz) measurement temperature: room temperature (22°C)
pulse mode: CPTOSS measurement
rotation number: 5000 Hz
pulse repeat time: 3 sec
contact time: 1 milli second
cumulated number: 2048 times
standard substance: glycine (outside standard: 176.03 ppm)

The ¹³C solid-state NMR spectrum was measured using Avance400 MHz (manufactured by Bruker) equipped with a 7 mm-CPMAS probe manufactured by Bruker, and according to the CPTOSS method (spinning side band elimination method) of carbon core (resonance frequency 100.6 MHz). A sample tube enclosing about 300 mg of a solid sample was rotated at 5 kHz, and measured at contact time 1 milli second, pulse waiting time 3 sec, cumulated number 2048 times, room temperature (22°C). The chemical shift was normalized by the outside standard method wherein the carbonyl carbon of glycine was 176.03 ppm.

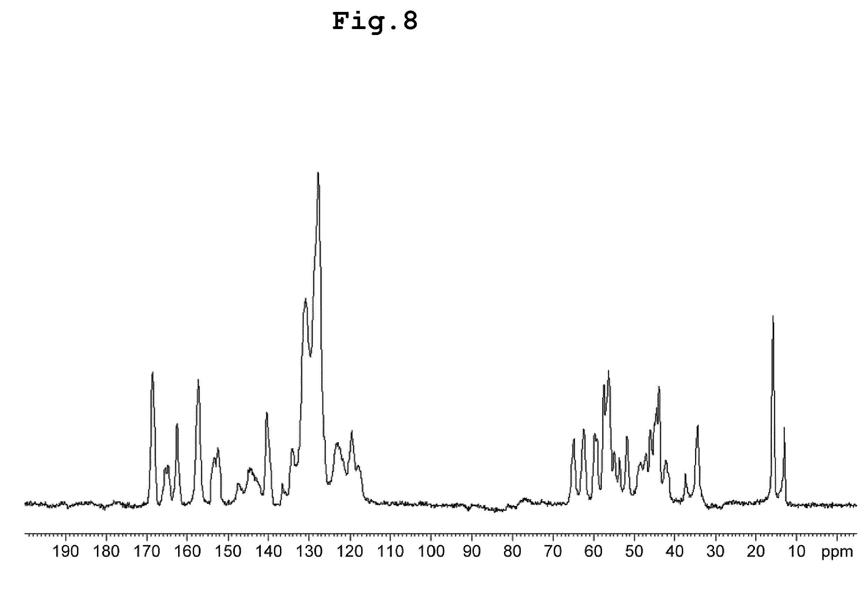

The ¹³C solid-state NMR spectrum of crystal A obtained in Example 1 is shown in Fig. 8. In Fig. 8, the ¹³C solid-state NMR spectrum of crystal A confirmed that it has peaks of chemical shifts (ppm) 15.8, 56.4, 127.8, 157.4 and 168.6.

## Claims

1. A crystal of 4-(((6S,9S)-1-(benzylcarbamoyl)-2,9-dimethyl-4,7-dioxo-8-(quinolin-8-ylmethyl)octahydro-1H-pyrazino[2,1-c][1,2,4]triazin-6-yl)methyl)phenyl dihydrogen phosphate or hydrate thereof.

2. A crystal of hydrate of 4-(((6S,9S)-1-(benzylcarbamoyl)-2,9-dimethyl-4,7-dioxo-8-(quinolin-8-ylmethyl)octahydro-1H-pyrazino[2,1-c][1,2,4]triazin-6-yl)methyl)phenyl dihydrogen phosphate.

3. The crystal according to claim 2, showing diffraction peak at diffraction angle (20±0.2') of 15.9°, in powder X-ray diffraction.

4. A crystal of hydrate of 4-(((6S,9S)-1-(benzylcarbamoyl)-2,9-dimethyl-4,7-dioxo-8-(quinolin-8-ylmethyl)octahydro-1H-pyrazino[2,1-c][1,2,4]triazin-6-yl)methyl)phenyl dihydrogen phosphate, showing peaks at chemical shifts (ppm) of 15.8, 56.4, 127.8, 157.4 and 168.6, in ¹³C solid-state NMR spectrum.

5. A production method of a crystal of 4-(((6S,9S)-1-(benzylcarbamoyl)-2,9-dimethyl-4,7-dioxo-8-(quinolin-8-ylmethyl)octahydro-1H-pyrazino[2,1-c][1,2,4]triazin-6-yl)methyl)phenyl dihydrogen phosphate or hydrate thereof, comprising a step of crystallizing from a solvent selected from the group consisting of an alcohol solvent, water and a combination of these.

6. A pharmaceutical composition comprising the crystal according to any one of claims 1 to 4 as an active ingredient.

7. An antitumor agent comprising the crystal according to any one of claims 1 to 4 as an active ingredient.

8. The antitumor agent according to claim 7, wherein the tumor is lung cancer, breast cancer, stomach cancer, pancreatic cancer, liver cancer, uterine cancer, ovarian cancer, glioma, melanoma, rectal colon cancer, lymphoma or blood cancer.
